**Europäisches Patentamt**

**European Patent Office**

**Office européen des brevets**

(19)

(11) Publication number : **0 326 547 B1**

# (12) EUROPEAN PATENT SPECIFICATION

(45) Date of publication of patent specification :
**15.04.92 Bulletin 92/16**

(51) Int. Cl.⁵ : **G01N 33/53**

(21) Application number : **87904632.4**

(22) Date of filing : **16.06.87**

(86) International application number :
**PCT/SE87/00281**

(87) International publication number :
**WO 88/00343 14.01.88 Gazette 88/02**

(54) REAGENT KIT, ITS USE AND DIAGNOSTIC PROCESS FOR THE DETECTION OF HPASP (HUMAN PANCREATIC SPECIFIC PROTEIN).

(30) Priority : **09.07.86 SE 8603050**

(43) Date of publication of application :
**09.08.89 Bulletin 89/32**

(45) Publication of the grant of the patent :
**15.04.92 Bulletin 92/16**

(84) Designated Contracting States :
**AT BE CH DE FR GB IT LI LU NL SE**

(56) References cited :
**US-A- 3 979 507
US-A- 4 446 240
CHEMICAL ABSTRACTS, vol.94, 1981, Columbus, OH (US); no.190140g
CHEMICAL ABSTRACTS, vol.92, 1980, Columbus, OH (US); no.108884e
CHEMICAL ABSTRACTS, vol.92, 1980, Columbus, OH (US); no.106132j**

(56) References cited :
**CHEMICAL ABSTRACTS, vol.83, 1975, Columbus, OH (US); no.5784g
CHEMICAL ABSTRACTS, vol.68, 1968, Columbus, OH (US); no.11207c**

(73) Proprietor : **DIAGNOSTIC PRODUCTS CORPORATION
5700 West 96th Street
Los Angeles California 90045 (US)**

(72) Inventor : **POUSETTE, Hans Ake Lennart
Svartlösavägen 226
S-125 31 Alvsjö (SE)**
Inventor : **CARLSTROM, Kjell Axel Martin
Pergsbergsbacken 11
S-123 43 Farsta (SE)**

(74) Representative : **Ellowicz, Leo et al
Octrooibureau Polak & Charlouis Laan Copes van Cattenburch 80
NL-2585 GD Den Haag (NL)**

EP 0 326 547 B1

## Description

This invention relates to a hitherto unknown human protein and its use for the demonstration of diseases associated with the organs in which said protein is present, predominantly in the pancreas. All the data obtained up to now indicate that the protein is highly specific for the human pancreas; for this reason the protein will be referred to below as "Human Pancreatic Specific Protein" = HPASP.

Pancreatic diseases are rather common, and cases of pancreatitis are often seen in emergency centers. A number of different proteins involved in food digestion have been characterized and employed as markers for pancreatic diseases (3, 4, 5). Similarly also the protein pattern of pancreatic juice has been characterized (1). But despite this fact there are only a very few commercially available tests (except amylase determination tests) which are helpful for diagnosing pancreatic diseases (2).

The routine test usually chosen for pancreatitis diagnosis is the test for determining amylase in serum, sometimes in combination with a test for pancreatic isoamylase determination. The amylase determination methods are well known, and they generally are both cheap and simple methods. They do have the drawback, however, that they are rather un-specific. Three out of ten patients suffering from abdominal pain and having increased serum amylase levels are found to have diseases that are unrelated to the pancreas (3); this clearly shows that there is a need for more specific diagnostic methods. To illustrate how urgently these methods are needed it may here be mentioned that 20 % of the patients finding their way into medical emergency centers have symptoms of pain in the abdomen, that is, symptoms where pancreatitis must always be considered to be one among several potential causes.

During the priority year the Swedish Patent Office has performed an International Type Search. The documents cited as being of particular relevance are references 13, 14 and 15. All three of them deal with pancreas specific proteins that are quite distinct from HPASP.

As some of the chief aspects of the invention may be mentioned reagent kits containing at least one protein preparation selected from among HPASP- and antiHPASP-antibody preparations, the use of the kit for forming (HPASP-antiHPASP) immune complex, and diagnostic methods for demonstrating abnormal conditions in HPASP-containing organs, chiefly in cases of pancreatitis and in other diseases involving destruction of pancreatic cells containing HPASP.

Subordinate aspects of the invention comprise HPASP preparations, HPASP determination methods, in particular immunochemical determination methods, and antibody preparationsso-called antiHPASP-antibody preparations directed against antigenic determinants which occur in HPASP and can be utilized for demonstrating the presence of HPASP in tissues and physiological fluids.

HPASP is characterized by its molecular weight being about 44,500 daltons (±5 %) as determined by SDS gel electrophoresis, while its isoelectric point is pH 6.9 (±5 %) and its amino acid composition is that shown in Table 1. These data of course apply to the analysis methods employed in the exemplification. The term "HPASP preparation" according to the invention comprises also various types of fragments and derivatives of the protein which possess at least one antigenic determinant that can be utilized in immunochemical determination of the protein. Thus HPASP according to the invention may be provided with one of the analytically detectable groups which are well known within the field of immunochemical assay methodology and which may be for instance radioactive, enzymatically active, fluorogenic, chemiluminogenic, biotinyl etc. groups. Also the HPASP may be bound to any of the various types of carrier molecules that are well known in immunochemical methodology and in immunosorbent contexts. Among the human proteins present in the HPASP preparations of this invention HPASP is usually the major component, that is, it amounts to >50 % (w/w).

Preparations enriched in HPASP can be produced in that the protein is extracted from human tissues or physiological fluids containing the protein, preferably from the pancreas. The most practical approach is by starting from pancreatic cytosol to purify the HPASP therefrom. A number of different biochemical separation methods may be utilized in the purification procedure, as e.g. electrophoresis, in the first place isoelectric focusing, centrifugation and/or liquid chromatography. This latter method comprises for instance high pressure liquid chromatography (HPLC), and gel, affinity and ion exchange chromatography. In particular with the aid of chromatofocusing HPASP can be obtained in a simple and quick manner, in high yield, and with a high degree of purity; but also the combination of gel chromatography (especially HPLC) and electrophoresis will produce a high purity form of the protein.

The invention comprises also antiHPASP-antibody preparations. This term means that the preparation is directed against HPASP and will not react immunochemically with other substances in a manner that would disturb a particular intended use. AntiHPASP-antibodies may be prepared in ways commonly employed for antibodies, by means of immunizing a suitable animal (for example rabbit, rat, mouse etc.) whereupon the thus resultant antibodies are worked up so as to obtain a suitable purity and form (derivatives, fragments etc.). The production of antiHPASP-antibodies may comprise also the so-called monoclonal technique. The antiHPASP-antibodies of the antibody preparation may thus be present in the form of an antiserum or may have been affinity-purified or be derivatized (e.g. may be covalently bound to some of the aforementioned types of analytically detectable groups, or may be chemically or physically bound to a so-called "solid phase", i.e. a phase that is insoluble in aqueous media)or they may have been fragmented into various different antiHPASP-antibody-active components, such as Fab, Fab′ or F(ab′)$_2$.

For certain variants of the invention, it is possible to utilize antibodies or HPASP in a so-called solid-phase-bound form. The binding of proteins to solid phases and using them in immunological determination methods belong to prior art technique (see for example ref. 12). As examples of solid phases may be mentioned particulate hydrophilic matrices which are swellable to form gels, are insoluble in water, and contain OH or NH$_2$ groups (e.g. polyamides, polysaccharides, poly/hydroxyalkylacrylates) and corresponding methacrylates etc.). The antibody or HPASP of the invention may be covalently or adsorptively bound to a water-insoluble matrix.

The antiHPASP-antibody preparation and HPASP preparation of this invention may be employed in vitro to form immune complexes containing at least one antiHPASP-antibody-active component immunochemically bound to HPASP. In such immune complexes, either HPASP or the antiHPASP component, or both, derive from a preparation according to the invention. The main fields in which this type of immune complex formation is utilized are (a) immunosorbent purification of HPASP or antiHPASP-antibodies, and (b) immunochemical determination procedures, preferably determination of HPASP. The invention is directed to both of these aspects, including also reagent kits therefor. Such reagent kits contain at least one preparation according to the invention.

For the formation of the immune complexes, a preparation of this invention is mixed (administered) with (to) a sample which contains an immunological counterpart to the species present in the preparation, said mixing/administration being effected under conditions such that the immune complex can be formed. This may be done in various ways. Either the sample is added to a preparation of the invention, or vice versa. Where a plurality of different preparations of the invention are employed, the order of their addition is determined by the contemplated use of the immune complex formed. The conditions are those that are commonly employed for immune reactions, i.e. aqueous media buffered to a pH normally within the range of from pH 3.5 to 9.0, preferably 5.0 to 8.6. Buffers and detergents, which are not detrimental to the immune reaction or its result, may be added.

In the case of that aspect of the invention which comprises immunosorbent purification of HPASP, solid-phase-bound antiHPASP-antibodies are utilized for the formation of immunocomplexes and then in their immunocomplexed form are separated from the reaction mixture, whereupon HPASP is liberated in a manner which is known per se in the context of immunosorbent purification techniques, the liberated HPASP being subjected to further work-up if required. In cases where antiHPASP-antibodies are to be purified, solid-phase-bound HPASP is employed in an analogous manner.

For HPASP determination in accordance with the diagnostic methods of this invention any analysis method may be employed which is capable of specifically distinguishing between HPASP and other proteins present in a sample. Thus for instance one may subject a serum sample to isoelectric focusing for separating HPASP from the remaining serum proteins, in order to then quantify the amount of protein in the HPASP fraction by means of protein analysis. A serum HPASP value thus found can be correlated to the pancreas function of the patient, a decreased value being indicative of a subfunctioning pancreas and an elevated value being indicative of pancreatitis or other abnormalities involving destruction of pancreatic cells containing HPASP, e.g. the destruction involved in the rejection of a transplanted pancreas.

However, the most practical methodology for clinical routine work is an immunochemical assay method. According to this method antibodies directed against HPASP are allowed to react with HPASP present in a sample to form an (HPASP-antiHPASP) immune complex the formation and amount of which are qualitative and quantitative measures, respectively, of the occurrence of HPASP in the sample. For facilitating detection and quantitation it is a fairly common practice to add further immune reactants or other reactants capable of reacting biospecifically with components in the complex. That may be HPASP of antiHPASP-antibodies which are bound to a solid phase or are provided with analytically detectable groups. If a labeled reactant is added the amounts of reactants are chosen such that the amount of labeled reactant binding or not binding to the immune complex will be indicative of the amount of analyte (HPASP) in the sample.

Various different immunochemical assay methods are available; the artisan will base his choice of the most suitable method on the occurrence of a given analyte. This applies also to HPASP. Among different methods may be mentioned: Competitive (inhibitions) and non-competitive methods, precipitation methods, heterogeneous and homogeneous methods, various methods denoted by the name of the analytically detectable group employed, immunoelectrophoresis, particle agglutination, immunodiffusion, and microscopy with the aid of labeled antibodies.

At the present stage we consider the so-called inhibition methods (competitive methods) to be the preferred methods for determining HPASP.

The reagent kit of the invention always contains HPASP or antiHPASP-antibody-active components, and these may form one, two or more preparations according to the invention, depending on the particular use contemplated.

If the kit is to be used for the purification of antiHPASP-antibody-active components it contains a preparation in which HPASP is bound to solid phase. If the kit is to be used for the purification of HPASP it contains antiHPASP-antibody-active components bound to solid phase.

If the kit is to be used for an immunochemical assay method it always contains an antiHPASP-antibody preparation. In case the method is one of the competitive type the kit may contain an HPASP preparation of the invention, in addition to antiHPASP-anti ody-active components optionally bound to solid phase. Depending on the competitive system to be utilized the immune reactants (HPASP and antiHPASP-antibody-active components) present in the kit may be either soluble or insoluble, labeled or unlabeled. If the method is a non-competitive method, as for instance a sandwich system, then the kit may contain two different kinds of antiHPASP-antibody-active components capable of being present substantially simultaneously in an immunologically HPASP-bound form. Depending on the particular method to be carried out the antibody components of the kit may be bound to solid phase, labeled with analytically detectable groups or unlabeled and insoluble.

Various aspects of the invention are set forth in the attached claims forming part of this text.

The invention will now be illustrated by the scientific work that has been conducive to this invention.

## MATERIALS AND METHODS

### Tissue samples

Human tissues were collected from corpses 24 to 28 hours after death, it having been duly ascertained that the donors had not suffered from any diseases affecting the organ to be analyzed. In some cases specimens were collected in connection with surgery. Following removal, tissues were frozen at -20 °C. After thawing they were homogenized in 3 volumes of ice-cold buffer A (Tris 50 mM, EDTA 1 mM, NaCl 50 mM, pH 7.4) with an Ultra-Turrax® homogenizer. The homogenate was centrifuged for 60 min at 100,500 x g, whereupon the floating lipid layer was removed and the remaining supernatant was decanted and stored at -70 °C until used.

### Serum samples

Blood was collected by venopuncture, the blood thus obtained being allowed to clot. Serum was then prepared by centrifugation at 3000 x g for 20 minutes. The serum was stored at -20 °C until it was analyzed.

### Protein and DNA determination

Determination of protein was performed according to the method of Lowry et al (6). In order to apply a proper amount of protein on the isoelectric focusing gels, the protein content of the HPLC eluate was determined from the absorbance difference at 280 and 310 nm.

DNA was analyzed according to Burton (7).

### High pressure liquid chromatography (= HPLC)

A Varian isoelectric model 5010 liquid chromatograph was kept in a cold room at 0 - 4 °C and used according to producer's instructions. The columns (SW 3000 (Varian)) were equilibrated in buffer A before use. Based on preliminary experiments two main principles were always used: (1) The columns were kept perpendicular, and (2) when two columns were employed they were placed parallel to each other and interconnected by means of a short tube of U shape. The eluate was collected in 0.5 ml fractions, care being taken that the dead space between the column and the fraction collector was kept at a minimum. The flow rate was set to 0.5 or 1 ml per minute giving an initial pressure of about 30 or 60 atm., depending upon whether one or two columns were used.

The following materials were employed to calibrate the column: Dextran blue (Pharmacia AB), void volume $2 \times 10^6$ dalton), human gamma-globulin (Sigma) MW 153,000 dalton, bovine serum albumin (Sigma) MW 67,000 dalton, carbonic anhydrase (Sigma) MW 30,000 dalton, myoglobin (Sigma) MW 17,500 dalton, RNase (Sigma) MW 13,700 dalton, cytochrome C (Sigma) MW 12,600 dalton and $CuSO_4$ (as free fraction). Protein concentration in the eluate fractions was measured on the basis of the difference in absorbance at 280 and 310 nm, and according to the values obtained aliquots were taken for further analysis. Routinely only fractions corresponding to the separation range of the column were collected, i.e. only fractions between Dextran blue and $CuSO_4$.

## Isoelectric focusing on polyacrylamide gel

Ready-made gels, pH 3.9 - 9 (LKB Produkter AB, Bromma, Sweden) were employed. Isoelectric focusing was performed with a Multiphor® or an Ultrophor® 2217 (LKB Produkter AB). Electrode strips soaked in 1 M NaOH (cathode) and 1 M sulfuric acid (anode) were applied, and the gel was prefocused for 15-20 minutes using a current of 15 mA. Sample frames 0.3 ml acrylic plastics were used for sample application. After prefocusing of the gel the sample frames were placed on the gel surface 0.8 - 1 cm from the cathode electrode strip where the pH was 8 - 8.5. Ferritin (pI 5.0) and hemoglobin (pI 7.4) dissolved in water and a mixture of commercially available standard proteins (Pharmacia AB, Uppsala, Sweden) were used as standards and were focused separately. Usually eight samples were focused simultaneously. Isoelectric focusing was initiated with a 50 mA current. The voltage was then increased stepwise to 1000 - 2000 V. When the ferritin standard was focused near the anode and the hemoglobin standard was focused in front of the sample frame (after about 1 hour), the liquid inside the sample frame was aspirated with a Pasteur pipette whereupon the sample frames were removed. The remaining sample solution on the gel was removed with a filter paper.

Focusing was continued until the current was constant. The total focusing time from the application of the sample was about 1.5 hour. Efficient cooling was of the utmost importance, and for this reason analyses were performed at 2 - 4 °C in that ice water was pumped all the time through the cooling plate of the apparatus. The pH was measured at the same temperature with the aid of a surface electrode (type 403-30, Ingold, Zurich, Switzerland).

## Protein dyeing

Gels were stained with Coomassie blue.

## Purification of HPASP

A pool of 200 ml of pancreatic cytosol was prepared in accordance with the procedure described under the heading "Tissue samples". Thirty ml were thawed on ice and dialyzed against starting buffer (0.025 M ethanolamine pH 8.6).

This material was added to a 10 ml chromatofocusing column (Mono P, Pharmacia AB, Sweden) equilibrated in starting buffer. After careful washing until no adsorbance was detectable at 280 and 310 nm the adsorbed material was eluted with 30 ml Polybuffer 74 (pH 6 - 7, Pharmacia AB), and the fractions corresponding to the major protein peak for pH 6.9 were pooled, further characterized and employed after lyophilization for the development of a radioimmuno-assay analysis method.

## Measurement of lipase and amylase activity

Samples of the fractions obtained on chromatofocusing the cytosol were examined in respect of amylase activity (CBR alpha amylase, Boehringer, Mannheim, Germany) and pancreatic lipase activity (Enzygnost® lipase, Behring, Germany).

## Polyacrylamide gel electrophoresis

Polyacrylamide gel electrophoresis was performed using a 10 % cross-linked gel both in the presence and in the absence of SDS (= sodium dodecyl sulfate). The electrode buffer had a pH of 8.6 (phosphate). When electrophoresis was carried out on radioactively labaled HPASP the gel (after electrophoresis) was cut into slices (width 8 mm) at right angles to the direction of electrophoresis. Each slice was introduced into a tube and the radioactivity of the slices was determined.

## Analysis of amino acid composition

HPASP from the chromatofocusing procedure was rechromatographed on Sephadex G-25 for achieving a complete change of buffer to 0.01 M sodium phosphate, pH 7.4. The amino acid composition was determined by means of a standard procedure at Central Amino Acid Analysis Laboratory (Biokemiska Inst., Uppsala, Sweden). Acid hydrolysis was employed, for a time of 24 hours and 72 hours. Threonine values and serine values were obtained by extrapolation to zero time of hydrolysis, to correct for incomplete hydrolysis. Methionine and cystine were determined after oxidation with performic acid. Tryptophan was analyzed after hydrolysis with mercaptoethanesulfonic acid.

## Preparation of $^{125}$I-labeled HPASP

Radioiodination was carried out by means of the Chloramine-T method (10). This gave preparations having specific radio-activities of from 100 to 260 Ci/g of protein.

## Immunization. Raising of antibodies

Four 2-year old mixed breed rabbits were immunized with pure HPASP by the procedure of Vaitukaitis et al (9). The protein, 100 µg in 500 µl of 0.9 % NaCl, was mixed with 1500 µl of Freund's complete adjuvant. This mixture was injected subcutaneously at 20 different sites on the back of the rabbits. Serum samples were taken at various times and tested for their capacity of binding $^{125}$I-labeled HPASP, by means of the radioimmunochemical method described below. After 2 months the animals were producing antisera of a titer sufficiently high for use in an immunochemical method.

## Radioimmunoassay

The phosphate buffer described by Wide et al (11) was used. Sheep anti-rabbit-gammaglobulin-coated particles (DASP, Organon N.V., Oss, Holland) were used at a concentration of one ampoule per 100 ml of buffer for the separation of HPASP bound to its homologous rabbit anti ody from non-bound HPASP. The antiserum was diluted 1:10,000. The standard curve comprised concentrations of from 5 up to 1000 µg pure protein per liter. $^{125}$I-labeled HPASP was employed at a concentration of about 100 µg per ml.

The total incubation mixture considered of 100 µl of $^{125}$I-labeled protein, 100 µl of standard or appropriately diluted sample, and 100 µl of antiserum. After incubation at room temperature for 2 hours each tube received an addition of 1 ml of suspension of particles coated with sheep antirabbit-gamma-globulin. The tubes were stoppered and rotated slowly at room temperature for another 16-20 hours. After centrifugation at 1000 x g for 3 min the supernatant was aspirated, the particles were washed twice with 0.9 % NaCl, and then the radioactivity bound to the particles was measured in a gamma counter.

## RESULTS

## High pressure liquid chromatography (HPLC)

This method was worked out using pools of cytosols prepared from 6 - 8 specimens of normal pancreas for each pool. HPLC gave an acceptable separation according to molecular weight. Experiments were made employing different amounts in the chromatography procedure. For analysis of pancreatic cytosol an amount of 50 or 100 µl was optimal, giving accurate separation on HPLC and enough material to be visualized by protein dyeing following isoelectric focusing. Among the columns tested, an optimal combination for pancreatic cytosol was provided by two 300 mm SW 3000 columns. Buffers containing higher salt concentrations were also tried, but as the eluates in these cases had to be desalted, low-concentration buffers were used routinely.

## Isoelectric focusing in polyacrylamide gels (= IFPAGE)

Isoelectric focusing is known to give an optimal resolution according to isoelectric point. The combination of HPLC and IFPAGE gave an excellent separation, and after protein dyeing different bands could be observed. The gels were usually preserved in view of the fact that no photographs could show the entire spectrum of the proteins. Among the visible proteins, the one having pI 6.9 was HPASP.

## Purification of HPASP

Following preliminary experiments the aforesaid purification method could be carried out and gave in one single step an almost homogeneous product. The protein eluted at pH 6.9 had a pI of 6.9. When an immunochemical determination method had been developed the cytosol preparation to be worked up was always checked in order to make sure that it had a high content of HPASP.

Homogeneity and amino acid composition of HPASP

A band was obtained upon electrophoresis on polyacrylamide gel, both in the presence and in the absence of SDS. In cases where HPASP had been radioactively labeled prior to electrophoresis all the radioactivity could be traced to the band corresponding to HPASP. The results showed the preparation to be homogeneous. The molecular weight could also be calculated from gel electrophoresis data, and was thus found to be 44,500 dalton (±5 %). The amino acid composition is set forth below (error range ±0.1 %).

Table 1

| | | | |
|---|---|---|---|
| Aspartic acid | 9.4 | Isoleucine | 2.3 |
| Threonine | 5.9 | Leucine | 10.7 |
| Serine | 5.1 | Norleucine | 0 |
| Glutamic acid | 14.2 | Tyrosine | 2.6 |
| Proline | 4.7 | Phenylalanine | 4.6 |
| Glycine | 3.0 | Histidine | 2.7 |
| Alanine | 7.9 | Lysine | 10.3 |
| Half-cystine | 5.1 | Tryptophan | 0.3 |
| Valine | 4.0 | Arginine | 4.1 |
| Methionine | 0.9 | Total | 98.7 |

(The error range mentioned above has to be ascribed chiefly to impurities potentially present in the preparation subjected to analysis.)

Assay for lipase and amylase activity

Purified HPASP did not show any amylase activity. Moreover, it was demonstrated that amylase activity and HPASP were separated by chromatofocusing. Serial dilutions of HPASP showed that less than 1 % of the material reacted immunochemically in an immunochemical assay for pancreatic lipase. Thus the contamination of lipase is less than 1 %.

Immunization and radioimmunoassay

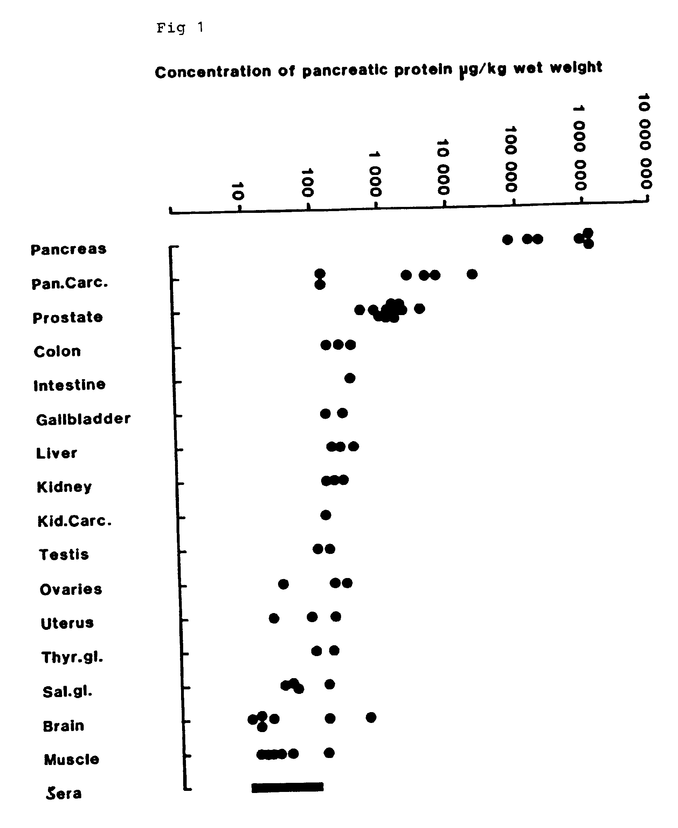

Serial dilution of human pancreatic cytosol gave a dose-response curve identical to the standard curve for HPASP. Parallel curves were also obtained when a pool of sera from patients suffering from acute pancreatitis was analyzed, High concentrations of HPASP in normal individuals could be detected only in the pancreas. A slightly elevated concentration as compared to serum could be demonstrated in the prostate. In all the other organs studied, only levels corresponding to peripheral serum levels could be demonstrated (Fig. 1).

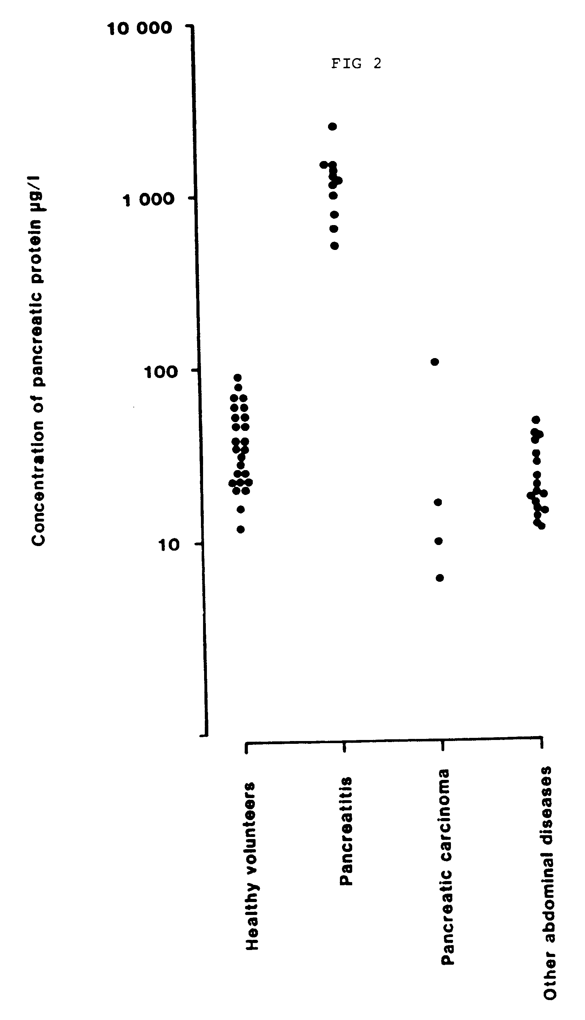

HPASP could be quantified in serum. In sera from 26 healthy individuals (13 of each sex) the level was 10 - 100 µg/l while patients suffering from acute pancreatitis had levels ten times higher (Fig. 2). Patients suffering from abdominal diseases not involving the pancreas did not show elevated levels.

During the priority year clinical studies have revealed that elevated serum levels of HPASP in pancreas transplanted patients might be a marker indicating the onset of rejection. Furthermore, patients having undergone surgical removal of pancreatic tissue have been shown to have decreased serum levels of HPASP indicating a correlation between subfunctioning pancreases and such levels.

Fig. 1: Concentration of HPASP in cytosol prepared from different human tissues. Each dot represents a value found in one individual.

Fig. 2: Concentration of HPASP in peripheral sera from 58 persons. Of these, 26 were healthy volunteers while the remainder of the sera had been collected at an emergency center. These latter sera were subdivided into various groups according to diagnoses.

REFERENCES

1. Scheele, G.A.
J. Biol, Chem. 14 (1975) 5375 - 5385.
2. Magid, E., Horsing, M., Rune, S.J.
Scand. J. Gastroent. 12 (1977) 621 - 627.
3. Skude, G., Eriksson, S.
Scand. J. Gastroenterol 11 (1976) 525 - 527.
4. Weaver, D., Douwman, D., Waltz, A., Clink, D., Resto, A., Stephany, J.
J. Surgery 92 (1982) 576 - 580.
5. Goodale, R., Condre, R., Dressel, T., Taylor T., Gajl-Pecjalska, K.
Ann. Surg. 189 (1979) 340 - 344.
6. Lowry, O.H., Rosebrough, N.J., Farr, A., Randall, R.J.
J. Biol. Chem. 193 (1951) 265 - 275.
7. Burton, K.
Meth. Enzymol. (Ed. Grossman and Moldave) 12B (1968) 163 - 166.
8. Pousette, Å., Carlström, K., Sköldefors, H., Wilking, N., Theve, N-O.
Cancer Res. 42 (1982) 633 - 637.
9. Vaitukaitis, I., Robbins, J.B., Nieschlag, E., Ross, G.T. J. Clin. Endocrinol. Metab. 33 (1971) 988 - 991.
10. Hunter, W.H., Greenwood, F.L.
Nature 194 (1962) 495 - 496.
11. Wide, L., Willius, S.J., Gemzell, C., Ross, P.
In: Radioimmunosorbent assay of follicle-stimulating hormone and luteinizing hormone in serum and urine from men and women.
Acta Endocrinol. (Kbh) 73 (1973) Suppl. 174, 11 - 15.
12. Wide, L et al
In: Radioimmunoassay and related Procedures in Medicine, Vol I (1978) 143-54.
13. Loor, R et al
Biochim Biophys Acta 668 (1981) 222-34.
14. US-A-4,446,240 (Nerenberg, S T).
15. US-A-3,979,507 (Baker, A L).

**Claims**

1. Substantially pure humanc pancreatic specific protein (=HPASP) having a molecular weight of 44,500 dalton and an isoeletric point of 6.9, having an amino acid composition which is set forth below (error range ± 0.1 %)

| | | | |
|---|---|---|---|
| Aspartic acid | 9.4 | Isoleucine | 2.3 |
| Threonine | 5.9 | Leucine | 10.7 |
| Serine | 5.1 | Norleucine | 0 |
| Glutamic acid | 14.2 | Tyrosine | 2.6 |
| Proline | 4.7 | Phenylalanine | 4.6 |
| Glycine | 3.0 | Histidine | 2.7 |
| Alanine | 7.9 | Lysine | 10.3 |
| Half-cystine | 5.1 | Tryptophan | 0.3 |
| Valine | 4.0 | Arginine | 4.1 |
| Methionine | 0.9 | | |

and being present in human pancreatic cytosol from which it can be isolated.

2. Antibody directed agaisnt determinants that are specific for human pancreatic specific protein (=HPASP) as claimed in claim 1.

8

3. Method for the diagnosis of a dysfunctioning pancreas in a human individual, wherein human pancreatic specific protein (=HPASP) according to claim 1 is determined in a sample of a biological fluid suspected of containing abnormal HPASP levels and derived from said individual, whereupon an abnormal level relative the normal population is taken as an indication for the individual having a pancreas that is dysfunctioning.

4. Method according to claim 3, wherein a decreased level relative the normal population is taken as an indication of a subfunctioning pancreas of the individual.

5. Method according to claim 3, wherein an increased level relative the normal population is taken as an indication for the individual suffering from acute pancreatitis.


**Patentansprüche**

1. Praktisch reines human-pankreasspezifisches Protein (=HPASP) mit einem Molekularge-wicht von 44.500 dalton und einem isoelektrischen Punkt von 6,9, mit einer Aminosäure-Mischung (Fehlerbereich +/- 0.1 %) wie unten angegeben:

| | | | |
|---|---|---|---|
| Asparaginsäure | 0,9 | Isoleucin | 2,3 |
| Threonin | 5,9 | Leucin | 10,7 |
| Serin | 5,1 | Norleucin | 0 |
| Glutaminsäure | 14,2 | Tyrosin | 2,6 |
| Prolin | 4,7 | Phenylalanin | 4,6 |
| Glycin | 3,0 | Histidin | 2,7 |
| Alanin | 7,9 | Lysin | 10,3 |
| Halb-Cystin | 5,1 | Tryptophan | 0,3 |
| Valin | 4,0 | Arginin | 4,1 |
| Methionin | 0,9, | | |

wobei es im human-pankreatischen Cytosol vorhanden ist, von dem es isoliert werden kann.

2. Antikörper, die gegen Determinanten gerichtet sind, die für human-pankreasspezifisches Protein nach Anspruch 1 spezifisch sind.

3. Verfahren zur Diagnose eines fehlerhaft funktionierenden Pankreas in einem menschlichen Individuum, wobei das human-pankreas-spezifische Protein (=HPASP) nach Anspruch 1 in einer Probe aus einem biologischen Fluidum, das in bezug auf abnormalen HPASP-Pegeln verdächtig ist und vom Individuum abgeleitet wurde, wonach ein abnormaler Pegel relativ zur normalen Bevölkerung als eine Indikation dafür genommen wird, dass das Individuum ein überfunktionierendes Pankreas hat.

4. Verfahren nach Anspruch 3, dadurch gekennzeichnet, dass der reduzierte Pegel von der normalen Bevölkerung als Indikation für eine Unterfunktion des Pankreas des Individuum genommen wird.

5. Verfahren nach Anspruch 3, dadurch gekennzeichnet, dass ein erhöhter Pegel relativ zur normalen Bevölkerung als Indikation für das Individuum genommen wird, das an einer akuten Pankreatitis leidet.


**Revendications**

1. Protéine spécifique au pancréas humain (=PSPH) sensiblement pure, ayant un poids moléculaire de 44.500 daltons et un point isolélectrique de 6,9 ainsi qu'une composition en acides aminés comme suit (plages d'erreurs de +/- 0,1 %) :

| | | | |
|---|---|---|---|
| Acide aspartique | 9,4 | Isoleucine | 2,3 |
| Thréonine | 5,9 | Leucine | 10,7 |
| Sérine | 5,1 | Norleucine | 0 |
| Acide glutamique | 14,2 | Tyrosine | 2,6 |
| Proline | 4,7 | Phénylalanine | 4,6 |
| Glycine | 3,0 | Histidine | 2,7 |
| Alanine | 7,9 | Lysine | 10,3 |
| Demi-cystine | 5,1 | Tryptophane | 0,3 |
| Valine | 4,0 | Arginine | 4,1 |
| Méthionine | 0,9 | | |

cette protéine étant présente dans le cytosol pancréatique humain à partir duquel elle peut être isolée.

2. Anticorps dirigé contre les déterminants qui sont spécifiques à la protéine spécifique au pancréas humain (PSPH) selon la revendication 1.

3. Procédé de diagnostic du mauvais fonctionnement du pancréas chez un être humain, dans lequel la protéine spécifique au pancréas humain (PSPH) suivant la revendication 1 est dosée dans un échantillon d'un fluide biologique suspecté de contenir des taux de PSPH anormaux et provenant de l'individu en question, un taux anormal relativement à la population normale indiquant que l'individu a un pancréas qui fonctionne mal.

4. Procédé selon la revendication 3, dans lequel un taux réduit relativement à la population normale est considéré comme l'indication d'un mauvais fonctionnement du pancréas de l'individu.

5. Procédé selon la revendication 3, dans lequel un taux accru relativement à la population normale est considéré comme l'indication que l'individu souffre d'une pancréatite aiguë.

Fig 1

Concentration of pancreatic protein µg/kg wet weight

FIG 2